(19) Europäisches Patentamt
European Patent Office
Office européen des brevets

(11) EP 0 720 655 B1

(12) **EUROPÄISCHE PATENTSCHRIFT**

(45) Veröffentlichungstag und Bekanntmachung des Hinweises auf die Patenterteilung:
**15.12.1999 Patentblatt 1999/50**

(21) Anmeldenummer: **94928365.9**

(22) Anmeldetag: **16.09.1994**

(51) Int Cl.[6]: **C12P 13/02**, C12P 41/00

(86) Internationale Anmeldenummer:
**PCT/EP94/03102**

(87) Internationale Veröffentlichungsnummer:
**WO 95/08636 (30.03.1995 Gazette 1995/14)**

(54) **RACEMATSPALTUNG PRIMÄRER UND SEKUNDÄRER AMINE DURCH ENZYM-KATALYSIERTE ACYLIERUNG**

RACEMATE CLEAVAGE OF PRIMARY AND SECONDARY AMINES BY ENZYME-CATALYSED ACYLATION

CLIVAGE DE RACEMATES D'AMINES PRIMAIRES ET SECONDAIRES PAR ACYLATION CATALYSEE PAR ENZYME

(84) Benannte Vertragsstaaten:
**AT BE CH DE DK ES FR GB IE IT LI NL PT SE**

(30) Priorität: **25.09.1993 DE 4332738**

(43) Veröffentlichungstag der Anmeldung:
**10.07.1996 Patentblatt 1996/28**

(73) Patentinhaber: **BASF Aktiengesellschaft
67063 Ludwigshafen (DE)**

(72) Erfinder:
- **BALKENHOHL, Friedhelm
D-67117 Limburgerhof (DE)**
- **HAUER, Bernhard
D-67136 Fussgoenheim (DE)**
- **LADNER, Wolfgang
D-67136 Fussgoenheim (DE)**
- **PRESSLER, Uwe
D-67122 Altrip (DE)**
- **NÜBLING, Christoph
D-67454 Ha loch (DE)**

(56) Entgegenhaltungen:
**EP-A- 0 443 406** **WO-A-90/14429**
**WO-A-91/19002** **US-A- 5 057 607**

- **CHEMICAL ABSTRACTS, vol. 115, no. 25, 23. Dezember 1991, Columbus, Ohio, US; abstract no. 278181j, Seite 847 ; & JP,A,3 191 797 (FUJI PHOTO FILM) 21. August 1991**
- **TETRAHEDRON, (INCL. TETRAHEDRON REPORTS), Bd.47, Nr.44, 11. November 1991, OXFORD GB Seiten 9207 - 9214 VICENTE GOTOT 'Enzymatic aminolysis and transamidation reactions.'**
- **JOURNAL OF THE CHEMICAL SOCIETY, CHEMICAL COMMUNICATIONS, Bd.20, Nr.2, 15. Oktober 1990, LETCHWORTH GB Seiten 1386 - 1387 ROSARIO BRIEVA ET AL. 'Enzymatic synthesis of amides with two chiral centres.' in der Anmeldung erwähnt**
- **TETRAHEDRON: ASYMMETRY, Bd.4, Nr.6, Juni 1993, OXFORD GB Seiten 1105 - 1112 MARGARITA QUIROS ET AL. 'Lipase-catalyzed synthesis of optically active amides in orgamic media.' in der Anmeldung erwähnt**
- **Angew. Chemie 101, 1989, 711 - 724**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein neues Verfahren zur Racematspaltung primärer und sekundärer Amine durch Umsetzung mit einem Ester in Gegenwart einer Lipase und anschließende Trennung des einen enantioselektiv acylierten Amins vom nicht umgesetzten anderen Enantiomer des Amins.

Die Racematspaltung von Aminen durch Enzym-katalysierte Umsetzung mit Estern ist bekannt. Kitaguchi et al. (J. Amer. Chem. Soc. 111, 3094-3095, 1989) beschreiben die Racematspaltung von Aminen mit Trifluorethylbutyrat unter Subtilisin-Katalyse. Die Enantioselektivität dieser Reaktion ist jedoch stark lösungsmittelabhängig. Selbst mit dem geeignetsten der beschriebenen Lösungsmittel (3-Methyl-3-pentanol) wird nur eine mäßige Selektivität erreicht.

In WO 91/19002 wird ein Verfahren zur chiralen Anreicherung von asymmetrischen primären Aminen beschrieben, bei dem die Amine unter Subtilisin-Katalyse mit Ethylacetat oder Ethylbutyrat umgesetzt werden. Die dabei erzielten Enantiomerenüberschüsse sind jedoch nicht befriedigend; außerdem werden lange Reaktionszeiten von einer bis mehreren Wochen benötigt.

Gotor et al. (J. Chem. Soc. Chem. Commun. 957-958, 1988) beschreiben die enantioselektive Acylierung von 2-Amino-butan-1-ol mit Ethylacetat unter Katalyse von Schweinepankreas-Lipase (PPL). Dabei wird der verwendete Ester (Ethylacetat) auch als Lösungsmittel eingesetzt. Bei Verwendung anderer Lösungsmittel bzw. anderer Enzyme werden keine befriedigenden Ergebnisse erzielt.

Von Brieva et al. (J. Chem. Soc. Chem. Commun., 1386-1387, 1990) wird die enantioselektive Synthese von Amiden aus racemischen primären Aminen durch Umsatz mit 2-Chlorpropionat unter Katalyse von Subtilisin in Hexan oder Candida cylindracea Lipase in 3-Methyl-3-pentanol beschrieben.

Quiros et al. (Tetrahedron: Asymmetry 4, 1105-1112, 1993) beschreiben die Lipase-katalysierte Synthese von optisch aktiven Amiden aus racemischen $\alpha$-Halogen-substituierten Propionsäureethylestern und primären Aminen. Die bei dieser Umsetzung erreichte Enantioselektivität kann jedoch nicht zufriedenstellen.

Asensio et al. (Tetrahedron Letters 32, 4197-4198, 1991) beschreiben die Lipase-katalysierte enantioselektive Acylierung von sekundären Aminen. Diese Reaktion verläuft jedoch nur bei einem Amin enantioselektiv und auch dort nur mit mäßigem Erfolg. Andere Amine zeigen überhaupt keine Enantioselektivität.

In US 5,057,607 wird ein Verfahren zur stereoselektiven Acylierung von primären Aminen mit Estern, die in Nachbarschaft zum Carbonylkohlenstoff ein Sauerstoffatom tragen, zur Synthese von $\beta$-Lactamen beschrieben. Als Katalysator im Verfahren wird die Penicillin-G-Amidase verwendet. Von Nachteil bei dieser Reaktion ist die geringe Substratbreite der Penicillin-G-Amidase, die als Edukte nur $\beta$-Lactam-Vorläufer akzeptiert.

Die bisher bekannten Verfahren zur enzymkatalysierten Racemattrennung sind entweder zu wenig enantioselektiv oder können nur unter ganz speziellen Bedingungen (Lösungsmittel, Enzym) durchgeführt werden. Außerdem benötigen sie für die Katalyse lange Reaktionszeiten und enorm große Enzymmengen, so daß ein darauf aufbauendes Verfahren nicht wirtschaftlich ist.

Es bestand daher die Aufgabe, ein Verfahren zur enzymkatalysierten Racematspaltung von primären und sekundären Aminen bereitzustellen, das eine hohe Enantioselektivität gewährleistet, in einem weiten Bereich von Reaktionsbedingungen eingesetzt werden kann und dabei mit möglichst geringen Mengen an Katalysator auskommt.

Demgemäß wurde gefunden, daß ein Verfahren zur Racematspaltung primärer und sekundärer Amine durch Umsetzung mit einem Ester unter spezifischer Katalyse mit einer Lipase und anschließende Trennung des einen, enantioselektiv acylierten Amins vom nicht umgesetzten anderen Enantiomer des Amins dann besonders gut funktioniert, wenn die Säurekomponente des Esters ein Fluor- oder Sauerstoffatom trägt, das an ein Kohlenstoffatom in $\alpha$-Position zum Carbonylkohlenstoff gebunden ist.

Weiterhin wurde ein Verfahren zur Herstellung von acylierten primären und sekundären Aminen durch Umsetzung der Amine mit einem Ester unter spezifischer Katalyse mit einer Lipase gefunden, das dadurch gekennzeichnet ist, daß die Säurekomponente des Esters ein Fluor- oder Sauerstoffatom trägt, das an ein Kohlenstoffatom in $\alpha$-Position zum Carbonylkohlenstoff gebunden ist.

Die für das erfindungsgemäße Verfahren geeigneten Ester sind solche, die in der Säurekomponente des Esters ein Fluor- oder Sauerstoffatom in Nachbarschaft zum Carbonylkohlenstoff tragen.

Unter Nachbarschaft zum Carbonylkohlenstoff ist die Bindung des Fluor- oder Sauerstoffatoms an ein Kohlenstoffatom in $\alpha$-Position zum Carbonylkohlenstoff zu verstehen. Als Heteroatom ist Sauerstoff bevorzugt.

Das Sauerstoffatom kann gegebenenfalls mit weiteren Gruppen, z.B. Alkylgruppen, verknüpft sein. In diesem Fall liegt eine Ethergruppe vor.

Die Alkoholkomponente des Esters spielt für das erfindungsgemäße Verfahren keine so entscheidende Rolle. Es können dafür verzweigte und unverzweigte $C_1$- bis $C_{10}$-Alkohole, die auch gegebenenfalls substituiert sein können, verwendet werden.

Besonders geeignete Ester sind solche mit der Struktur

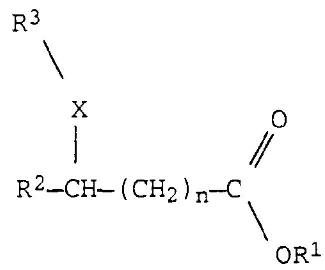

worin

$R^1$ = $C_1$-$C_{10}$-Alkyl,
$R^2$ = $C_1$-$C_{10}$-Alkyl, H,
$R^3$ = H, $C_1$-$C_{10}$-Alkyl, gegebenenfalls durch $NH_2$, OH, $C_{1-4}$-Alkoxy oder Halogen substituiertes Phenyl,
X = O
n = 0

bedeuten. Unter diesen sind die $C_{1-4}$-Alkylester der $C_{1-4}$-Alkoxyessigsäuren, wie der Methoxyessigsäureethylester, bevorzugt.

Im erfindungsgemäßen Verfahren werden als Katalysatoren Lipasen verwendet. Als Lipasen sind vor allem mikrobielle Lipasen gut geeignet, die beispielsweise aus Hefen oder Bakterien isolierbar sind. Besonders gut geeignet sind Lipasen aus Pseudomonas, z. B. Amano P oder die Lipase aus Pseudomonas spec. DSM 8246. Des weiteren können die Lipasen "Chirazyme L1 bis L8', welche kommerziell erhältlich sind (Boehringer Mannheim), vorteilhaft in dem erfindungsgemäßen Verfahren eingesetzt werden.

Das verwendete Enzym kann in nativer oder in unmobilisierter Form eingesetzt werden.

Als Lösungsmittel sind generell organische Lösungsmittel geeignet. Besonders gut verläuft die Reaktion in Ethern, beispielsweise in MTBE oder THF, oder in Kohlenwasserstoffen wie Hexan, Cyclohexan, Toluol oder halogenierten Kohlenwasserstoffen wie Methylenchlorid.

Als primäre und sekundäre Amine können auch Aminoalkohole eingesetzt werden.

Die Umsetzung des Esters mit dem racemischen Amin bzw. Aminoalkohol unter Enzymkatalyse wird üblicherweise bei Raumtemperatur ausgeführt. Die Reaktionszeiten dafür betragen je nach Substrat 1 bis 48 Stunden. Sekundäre Amine/Aminoalkohole benötigen in der Regel längere Reaktionszeiten als primäre Amine/Aminoalkohole. Die geringere Reaktivität sekundärer Amine kann auch durch eine gegenüber primären Aminen erhöhte Menge an Katalysator ausgeglichen werden.

Pro Mol umzusetzendes Substrat werden 1 bis 3 Mol Ester zugesetzt. Auch bei Verwendung racemischer Substrate werden 1 bis 3 Mol Ester zugesetzt.

Die zuzusetzende Menge an Enzym hängt von der Art der Lipase und der Aktivität der Enzympräparation ab. Die für die Reaktion optimale Enzymmenge kann leicht durch einfache Vorversuche ermittelt werden. In der Regel werden 1000 Units Lipase pro mMol Amin bzw. Aminoalkohol zugesetzt.

Der Reaktionsverlauf läßt sich leicht mit üblichen Methoden beispielsweise mittels Gaschromatographie verfolgen. Im Falle der Racematspaltung beendet man die Reaktion sinnvollerweise bei einem Umsatz von 50% des racemischen Amins bzw. Aminoalkohols. Dies geschieht in der Regel durch Entfernen des Katalysators aus dem Reaktionsraum, beispielsweise durch Abfiltrieren des Enzyms.

Durch die enantioselektive Umsetzung des racemischen Substrats mit dem Ester entsteht aus dem einen Enantiomer das entsprechend acylierte Produkt (Amid), während das andere Enantiomer unverändert bleibt. Das nun vorliegende Gemisch aus Amin und Amid läßt sich mit üblichen Methoden leicht trennen. Gut geeignet zur Trennung des Gemisches aus Amin und Amid sind beispielsweise Extraktions- oder Destillationsverfahren.

Das erfindungsgemäße Verfahren eignet sich zur Acylierung von allen primären und sekundären Aminen. Auch die Racematspaltung von praktisch allen primären und sekundären Aminen ist damit durchführbar. Besonders gut verläuft es bei primären Arylalkylaminen, beispielsweise solchen mit folgenden Strukturen:

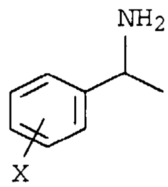

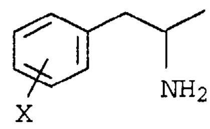

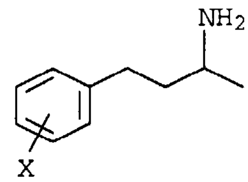

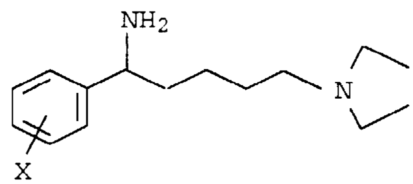

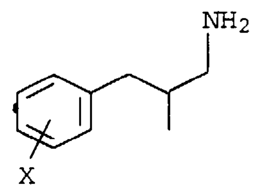

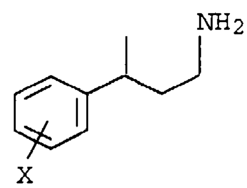

wobei X für alle gängigen Aromatensubstituenten, insbesondere Halogen, Nitro, Cyano, $C_1$-$C_4$-Alkyl, $C_1$-$C_4$-Alkoxy und $C_1$-$C_4$-Alkylthio, steht.

Des weiteren eignet sich das erfindungsgemäße Verfahren zur enantioselektiven Acylierung von Aminoalkoholen der allgemeinen Formel

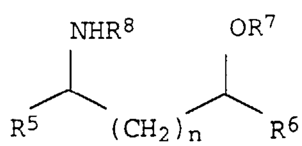

in der die Substituenten folgende Bedeutung haben:

$R^5$ $R^6$ = unabhängig voneinander H, verzweigtes und unverzweigtes $C_1$-$C_{10}$-Alkyl, $C_1$-$C_4$-Alkoxycarbonyl, Phenyl, Phenyl-$C_1$-$C_4$-alkyl, wobei die Phenylgruppen durch Halogen, Nitro, Cyano, $C_1$-$C_4$-Alkyl, $C_1$-$C_4$-Alkoxy und $C_1$-$C_4$-Alkylthio substituiert sein können. Darüber hinaus können $R^5$ und $R^6$ über eine Kohlenstoffkette, die durch Sauerstoff, Schwefel oder Stickstoff unterbrochen und ihrerseits substituiert sein kann, zu einem Mono-, Bi- oder Tricyclus geschlossen sein

$R^7$ = H, $C_1$-$C_{10}$-Alkyl, $C_1$-$C_4$-Alkoxycarbonyl

$R^8$ = H, $C_1$-$C_{10}$-Alkyl

n = 0 oder 1.

Sofern die durch $OR^7$ bzw. $NHR^8$ substituierten Kohlenstoffatome stereogene Zentren sind, bezieht sich das erfindungsgemäße Verfahren sowohl auf die syn- als auch die anti-Isomeren.

Als Beispiele für Aminoalkohole der obigen allgemeinen Struktur seien genannt:
2-Amino-1-butanol; Ephedrin; Pseudoephedrin; Norephedrin; Norpseudoephedrin; tert. Leucinol; Phenylglycidol; 1,2-Diphenylaminoethanol; cis- und trans-2-Aminocyclopentanol; cis- und trans-1-Amino-2-hydroxyindan; cis- und trans-2-Aminocyclohexanol, Statin, 2-Hydroxy-3-amino-phenylpropionsäure.

Als bevorzugte Aminoalkohole sind zu nennen: cis- und trans-1-Amino-2-hydroxyindan.

Die Erfindung eignet sich auch zur Herstellung von optisch aktiven primären und sekundären Aminen aus den entsprechenden Racematen, in dem man

a) ein racemisches Amin bzw. einen racemischen Aminoalkohol mit einem Ester, dessen Säurekomponente ein Fluor- oder Sauerstoffatom in Nachbarschaft zum Carbonylkohlenstoffatoms trägt, in Gegenwart einer Lipase enantioselektiv acyliert,

b) das Gemisch aus optisch aktivem Amin und optisch aktivem acylierten Amin trennt und somit ein Enantiomer des Amins erhält,

c) gewünschtenfalls aus dem acylierten Amin das andere Enantiomere des Amins bzw. Aminoalkohols durch Amidspaltung gewinnt.

Das erfindungsgemäße Verfahren läßt sich noch ökonomischer gestalten, wenn man nach Abtrennung des gewünschten Enantiomers das verbliebene, nicht gewünschte Enantiomer racemisiert und erneut in das Verfahren einsetzt. Durch diese Rückführung wird es möglich, insgesamt mehr als 50% des gewünschten Enantiomers aus dem racemischen Amin zu erhalten.

Die erfindungsgemäßen Verfahren eignen sich nicht nur als Herstellverfahren zur Produktion optisch aktiver primärer und sekundärer Amine und Aminoalkohole, sondern können auch Bestandteil von komplizierten chemischen Mehrstufensynthesen, beispielsweise bei der Herstellung von Arzneiwirkstoffen oder Pflanzenschutzmitteln, sein.
Die folgenden Beispiele dienen der Veranschaulichung der Erfindung.

Beispiel 1: Allgemeine Arbeitsvorschrift Lipase-katalysierte Acylierung von Aminen

10 mmol des primären oder sekundären Amins werden in MTBE (= Methyl-tert.butylether) gelöst (ca. 10 %ige Lösung). Die Lösung wird mit 11 mmol Methoxyessigsäureethylester versetzt und die Reaktion durch Zusatz von 100 mg Lipase (ca. 1000 U/mg, Pseudomonas spec. DSM 8246) gestartet. Bei vollständigem Umsatz (je nach Amin 12 bis 48 h) wird das Enzym abfiltriert und die Lösung im Vakuum konzentriert. Man erhält die Methoxyacetamide in einer Ausbeute von über 90 Prozent.

Eingesetzte Amine:

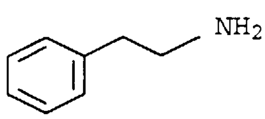

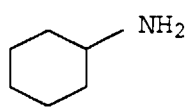

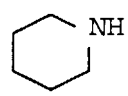

Beispiel 2: Allgemeine Arbeitsvorschrift für Racematspaltung

Das primäre oder sekundäre Amin wird in MTBE gelöst (ca. 10 Gew.%). Nach Zusatz von 1 Mol Methoxyessigsäureethylester pro 1 Mol racemisches Amin wird Pseudomonas-Lipase (DSM 8246) zugesetzt und die Suspension bei Raumtemperatur gerührt. Pro mMol Amin werden etwa 10000 Units Lipase (10mg) zugesetzt. Nach Erreichen eines Umsatzes von 50% (Überprüfung mittels Gaschromatographie), was je nach Amin nach 1-48 h erreicht ist, wird das Enzym abfiltriert. Das Gemisch aus Amin und acyliertem Amin (Amid) wird durch Destillation oder Extraktion getrennt.

Beispiel 3: Racematspaltung

Gemäß Beispiel 2 wurden 20 g (1) mit 1 Äquivalent (2) in Gegenwart von 2 g Pseudomonas-Lipase in MTBE bei Raumtemperatur umgesetzt. Nach 22 h war ein Umsatz von 50 % erreicht.

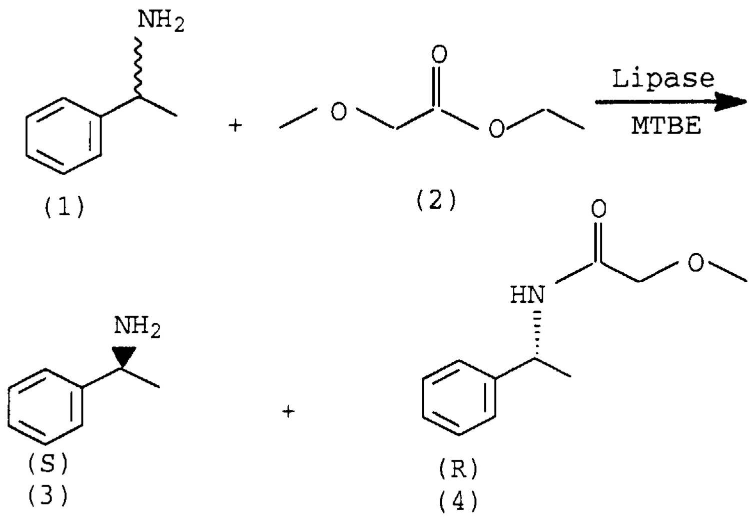


(1) (2)

(S) (3) (R) (4)

Die Ausbeute an (3) betrug 45 % (ee > 99 %),
die Ausbeute an (4) betrug 45 % (ee > 90 %).

Beispiel 4: Racematspaltungen

Gemäß Beispiel 2 wurden verschiedene Racematspaltungen durchgeführt. Es wurden verschiedene Amine bei unterschiedlichen Reaktionsbedingungen eingesetzt. Die Einzelheiten sind der Tabelle zu entnehmen.

Tabelle

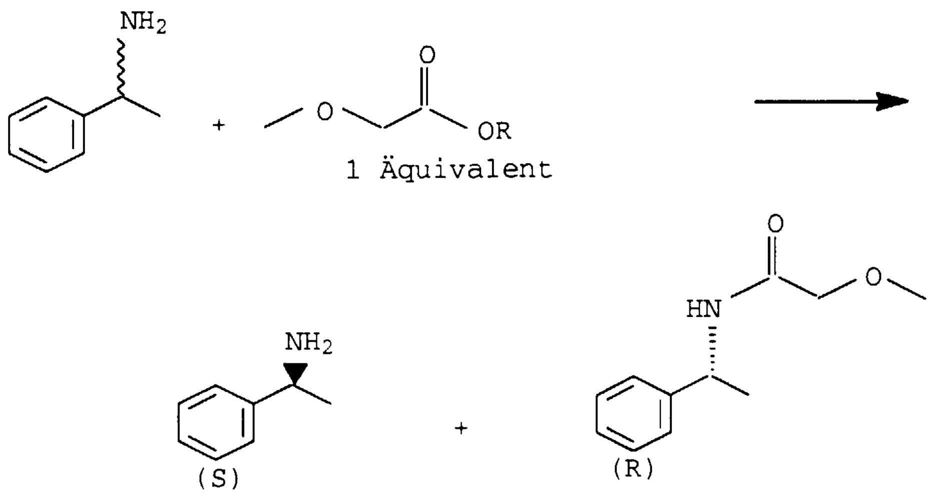


(S) (R)

| Nr. | racemisches Amin (g) | R | MTBE (ml) | Lipase (g;U/mg) | Zeit (h) | Umsatz per GC (%) | ee (Amin) (%) | ee (Amid) (%) |
|---|---|---|---|---|---|---|---|---|
| 1 | 20 | Et | 180 | 2; 1000 | 7<br>72 | 47<br>57 | > 99 | 93 |
| 2 | 5 | Et | 45 | 0,5;1000 | 3<br>22 | 43,6<br>56,1 | 95 | 96 |
| 3 | 5 | Me | 45 | 0,5;1000 | 23<br>23 | 38,5<br>51,4 | 79 | 97 |
| 4 | 2,5 | Et | 20 | 0,5;1000 | 1<br>3<br>24<br>48 | 39,6<br>50,1<br>59<br>59 | > 99 | 87 |
| 5 | 2,5 | Et | 20 | 0,5;1000 | 1<br>4 | 41<br>52 | 85 | 97 |
| 6 | 2,5 | Et | 25 | 0,125;1000 | 27 | 56,1 | 87 | 97 |
| 7 | 2,5 | Et | 25 | 0,5;1000 | 21 | 60,1 | 93 | 98 |
| 8 | 2,5 | Et | 10 | 0,25;1000 | 22 | 50,6 | 82 | 98 |
| 9 | 2,5 | Et | 25 | 0,25;1000 | 21 | 55,7 | 87 | 99 |
| 10*) | 2,5 | Et | 25 | 0,25;1000 | 4,5 | 40 | 50 | 97 |
| 11**) | 2,5 | Et | 25 | 0,25;1000 | 21 | 56,1 | 92 | 98 |
| 12 | 2,5 | Et | 25 | 1; 200 | 2 | 56,1 | 67 | > 99 |
| 13 | 2,5 | Me | 25 | 0,5; 200 | 4 | 54,3 | 69 | 99 |
| 14 | 2,5 | Bu | 25 | 0,5; 200 | 4 | 54,2 | 68 | > 99 |
| 15***) | 2,5 | Et | 25 | 0,5; 200 | 3 | 54,8 | 57 | > 99 |
| 16 | §) Amin 1 | Et | 20 | 0,5;1000 | 15,5 | 56 | 48 | 52 |
| 17 | §) Amin 2 | Et | 20 | 0,5;1000 | 3 | 53,6 | 70 | 75 |
| 18 | §) Amin 3 | Et | 25 | 0,25;1000 | 2 | 53,9 | 83 | > 99 |
| 19 | §) Amin 4 | Et | 25 | 0,5;1000 | 1,5 | 54,6 | 97 | > 99 |

*) wie Versuch Nr. 9 jedoch bei 50°C Reaktionstemperatur

**) Der Reaktionsansatz wurde geschüttelt anstatt gerührt

***) Als Ester wurde Butoxyessigsäureethylester anstatt Methoxyessigsäureester eingesetzt.

§) jeweils 20,6 mMol

Amin 1: 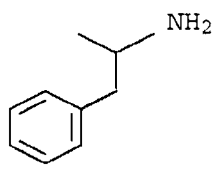

Amin 2: 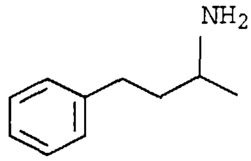

Amin 3: 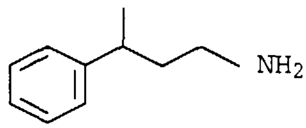

Amin 4: 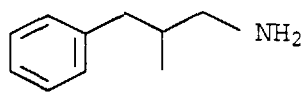

Beispiel 5: Racematspaltung von Aminoalkoholen

a) Eine Suspension von 150 mg (1 mmol) trans-1-Amino-2-hydroxyindan und 350 mg (3 mmol) Methoxyessigsäureethylester in 10 ml Methyl-tert.butylether wurde mit 9,6 mg "Chirazyme L2" versetzt und die Mischung bei Raumtemperatur gerührt. Nach 41 h war ein Umsatz von 49 % erreicht. Nach Zugabe von 3 ml Ethanol wurde das Enzym abfiltriert, das Filtrat zur Trockne eingeengt und der Rückstand in 10 ml 1N HCl aufgenommen. Nach Extraktion ($CH_2Cl_2$), Trocknung ($M_9SO_4$) der organischen Phase und Einengen wurde das Hydroxyamid in Form weißer Kristalle erhalten.

Rohausbeute: 100 mg (42%),
$[\alpha]_D^{20}$= -53,9° (c = 1,65; $CH_2Cl_2$), ec > 95 %.

b) Analog Beispiel 5a) wurden 150 mg (1 mmol) cis-1-Amino-2-hydroxyindan mit 350 mg (3 mmol) Methoxyessigsäureethylester und 50 mg "Chirazyme L1" umgesetzt. Nach 48 h war ein Umsatz von 50 % erreicht. Der Enantiomerenüberschuß des analog obiger Aufarbeitung erhaltenen Hydroxyamids betrug 71 %.

**Patentansprüche**

**1.** Verfahren zur Herstellung von acylierten primären und sekundären Aminen durch Umsetzung der Amine mit einem Ester in Gegenwart einer Lipase, dadurch gekennzeichnet, daß die Säurekomponente des Esters ein Fluor- oder Sauerstoffatom trägt, das an ein Kohlenstoffatom in α-Position zum Carbonylkohlenstoff gebunden ist.

**2.** Verfahren zur Racematspaltung primärer und sekundärer Amine durch Umsetzung mit einem Ester in Gegenwart einer Lipase und anschließende Trennung des einen, enantioselektiv acylierten Amins vom nicht umgesetzten anderen Enantiomer des Amins, dadurch gekennzeichnet, daß die Säurekomponente des Esters ein Fluor- oder Sauerstoffatom trägt, das an ein Kohlenstoffatom in α-Position zum Carbonylkohlenstoff gebunden ist.

**3.** Verfahren zur Herstellung von optisch aktiven primären und sekundären Aminen aus den entsprechenden Racematen, dadurch gekennzeichnet, daß man

a) ein racemisches Amin mit einem Ester, dessen Säurekomponente ein Fluor- oder Sauerstoffatom trägt, das an ein Kohlenstoffatom in α-Position zum Carbonylkohlenstoff gebunden ist, in Gegenwart einer Lipase enantioselektiv acyliert,

b) das Gemisch aus optisch aktivem Amin und optisch aktivem acylierten Amin trennt und somit ein Enantiomer des Amins erhält,

c) gewünschtenfalls aus dem acylierten Amin das andere Enantiomere des Amins durch Amidspaltung gewinnt.

**4.** Verfahren gemäß Anspruch 3, dadurch gekennzeichnet, daß man in Anschluß an Schritt b) oder c) in einem weiteren Schritt das nicht-gewünschte Enantiomer racemisiert und anschließend in das Verfahren der Racematspaltung zurückführt.

**5.** Verfahren zur Herstellung von optisch aktiven Verbindungen, dadurch gekennzeichnet, daß es mindestens als Teilschritt ein Verfahren nach Anspruch 2 bis 4 beinhaltet.

**6.** Verfahren nach Anspruch 2 oder 3, dadurch gekennzeichnet, daß als primäres oder sekundäres Amin ein Aminoalkohol eingesetzt wird.

**7.** Verfahren nach Anspruch 6, dadurch gekennzeichnet, daß der Aminoalkohol cis- oder trans-1-Amino-2-hydroxyindan ist.

**8.** Verfahren nach Anspruch 6, dadurch gekennzeichnet, daß der Aminoalkohol die Formel

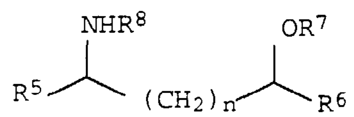

aufweist, in der die Substituenten folgende Bedeutung haben:

| | |
|---|---|
| $R^5$ $R^6$ = | unabhängig voneinander H, verzweigtes und unverzweigtes $C_1$-$C_{10}$-Alkyl, $C_1$-$C_4$-Alkoxycarbonyl, Phenyl, Phenyl-$C_1$-$C_4$-alkyl, wobei die Phenylgruppen durch Halogen, Nitro, Cyano, $C_1$-$C_4$-Alkyl, $C_1$-$C_4$-Alkoxy und $C_1$-$C_4$-Alkylthio substituiert sein können, oder $R^5$ und $R^6$ können über eine Kohlenstoffkette, die durch Sauerstoff, Schwefel oder Stickstoff unterbrochen und ihrerseits substituiert sein kann, zu einem Mono-, Bi- oder Tricyclus geschlossen sein; |
| $R^7$ = | H, $C_1$-$C_{10}$-Alkyl, $C_1$-$C_4$-Alkoxycarbonyl; |
| $R^8$ = | H, $C_1$-$C_{10}$-Alkyl; |
| n = | 0 oder 1. |

**9.** Verfahren nach einem der vorstehenden Ansprüche, dadurch gekennzeichnet, daß als Ester ein $C_1$-$C_4$-Alkylester einer $C_1$-$C_4$-Alkoxyessigsäure eingesetzt wird.

## Claims

**1.** A process for preparing acylated primary and secondary amines by reacting the amines with an ester in the presence of a lipase, wherein the acid component of the ester has a fluorine or oxygen atom which is bonded to a carbon atom in the a position relative to the carbonyl carbon.

**2.** A process for resolving racemates of primary and secondary amines by reacting with an ester in the presence of a lipase and subsequently separating one amine which has been enantioselectively acylated from the other, unreacted, enantiomer of the amine, wherein the acid component of the ester has a fluorine or oxygen atom which is bonded to a carbon atom in the α position relative to the carbonyl carbon.

**3.** A process for preparing optically active primary and secondary amines from the corresponding racemates, which comprises

a) enantioselectively acylating a racemic amine with an ester whose acid component has a fluorine or oxygen atom which is bonded to a carbon atom in the α position relative to the carbonyl carbon, in the presence of a lipase,

b) separating the mixture of optically active amine and optically active acylated amine and thus obtaining one enantiomer of the amine,

c) if required obtaining the other enantiomer of the amine from the acylated amine by amide cleavage.

**4.** A process as claimed in claim 3, wherein step b) or c) is followed by another step in which the unwanted enantiomer is racemized and then returned to the process for resolving the racemate.

**5.** A process for preparing optically active compounds, which comprises at least in part a process as claimed in claims 2 to 4.

**6.** A process as claimed in claim 2 or 3, wherein the primary or secondary amine used is an amino alcohol.

**7.** A process as claimed in claim 6, wherein the amino alcohol is cis- or trans-1-amino-2-hydroxyindane.

**8.** A process as claimed in claim 6, wherein the amino alcohol has the formula

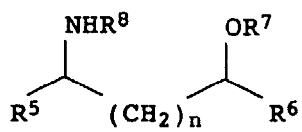

where

| | |
|---|---|
| $R^5$, $R^6$ | = independently of one another H, branched and unbranched $C_1$-$C_{10}$-alkyl, $C_1$-$C_4$-alkoxycarbonyl, phenyl, phenyl-$C_1$-$C_4$-alkyl, it being possible for the phenyl groups to be substituted by halogen, nitro, cyano, $C_1$-$C_4$-alkyl, $C_1$-$C_4$-alkoxy and $C_1$-$C_4$-alkylthio, or $R^5$ and $R^6$ may be closed to a mono-, bi- or tricyclic system by a carbon chain which may be interrupted by oxygen, sulfur or nitrogen and in turn substituted; |
| $R^7$ | = H, $C_1$-$C_{10}$-alkyl, $C_1$-$C_4$-alkoxycarbonyl; |
| $R^8$ | = H, $C_1$-$C_{10}$-alkyl; |
| n | = 0 or 1. |

**9.** A process as claimed in any of the preceding claims, wherein the ester used is a $C_1$-$C_4$-alkyl ester of a $C_1$-$C_4$-alkoxyacetic acid.

## Revendications

**1.** Procédé pour la préparation d'amines primaires et secondaires acylées par réaction des amines avec un ester en présence d'une lipase, caractérisé par le fait que le composant acide de l'ester porte un atome de fluor ou d'oxygène relié à un atome de carbone en position α du carbone de carbonyle.

**2.** Procédé pour la résolution de racémates d'amines primaires et secondaires par réaction avec un ester en présence d'une lipase suivie de la séparation de l'une des amines, acylée énantiosélectivement, d'avec d'autre anantiomère de l'amine, non converti, caractérisé par le fait que le composant acide de l'ester porte un atome de fluor ou d'oxygène relié à un atome de carbone en position alpha du carbone de carbonyle.

**3.** Procédé pour la préparation d'amines primaires et secondaires à l'état d'isomères optiques à partir des racémates correspondants, caractérisé par le fait que

a) on acyle énantiosélectivement une amine racémique par un ester dont le composant acide porte un atome de fluor ou d'oxygène relié à un atome de carbone en position alpha du carbone de carbonyle, en présence

d'une lipase,

b) on sépare le mélange de l'amine à l'état d'isomère optique et de l'amine acylée à l'état d'isomère optique, ce qui permet d'isoler un énantiomère de l'amine,

c) si on le désire, on obtient, à partir de l'amine acylée, l'autre énantiomère de l'amine par scission de l'amide.

**4.** Procédé selon la revendication 3, caractérisé par le fait que, après le stade b) et c), et dans un autre stade opératoire, on racémise l'énantiomère non recherché puis on le recycle au procédé de résolution des racémates.

**5.** Procédé pour la préparation de composés à l'état d'isomères optiques, caractérisé par le fait qu'il comporte, au moins en tant que stade partiel, un procédé selon les revendications 2 à 4.

**6.** Procédé selon la revendication 2 ou 3, caractérisé par le fait que l'amine primaire ou secondaire mise en oeuvre est un aminoalcool.

**7.** Procédé selon la revendication 6, caractérisé par le fait que l'aminoalcool est le cis- ou le trans-1-amino-2-hydroxy-indane.

**8.** Procédé selon la revendication 6, caractérisé par le fait que l'aminoalcool répond à la formule

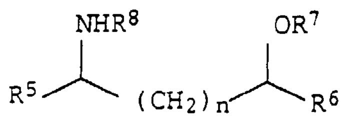

dans laquelle les symboles ont les significations suivantes :

$R^5$ $R^6$ = indépendamment l'un de l'autre, H, un groupe alkyle à chaîne droite ou ramifiée en C1-C10, un groupe (alcoxy en C1-C4)carbonyle, phényle, phényl-alkyle en C1-C4, les groupes phényle pouvant eux-mêmes être substitués par des halogènes, des groupes nitro, cyano, alkyle en C1-C4, alcoxy en C1-C4 et alkylthio en C1-C4, ou bien $R^5$ et $R^6$ peuvent être reliés entre eux en un mono-, un bi- ou un tri-cycle par l'intermédiaire d'une chaîne carbonée qui peut elle-même être interrompue par l'oxygène, le soufre ou l'azote et qui peut être substituée ;

$R^7$ = H, alkyle en C1-C10, (alcoxy en C1-C4)carbonyle ;

$R^8$ = H, alkyle en C1-C10 ;

n = 0 ou 1.

**9.** Procédé selon l'une des revendications qui précèdent, caractérisé par le fait que l'ester mis en oeuvre est un ester alkylique en C1-C4 d'un acide (alcoxy en C1-C4)acétique.